**Europäisches Patentamt**

**European Patent Office**

**Office européen des brevets**

(19)

(11) Veröffentlichungsnummer: **0 036 577**
**B1**

(12)

# EUROPÄISCHE PATENTSCHRIFT

(45) Veröffentlichungstag der Patentschrift:
20.06.84

(51) Int. Cl.³: **G 10 K 11/34**

(21) Anmeldenummer: **81101841.5**

(22) Anmeldetag: **12.03.81**

(54) **Ultraschall-Array.**

(30) Priorität: **17.03.80 DE 3010210**

(43) Veröffentlichungstag der Anmeldung:
**30.09.81 Patentblatt 81/39**

(45) Bekanntmachung des Hinweises auf die Patenterteilung:
**20.06.84 Patentblatt 84/25**

(84) Benannte Vertragsstaaten:
**AT CH DE FR GB LI NL**

(56) Entgegenhaltungen:
**EP - A - 0 021 534**
**DE - A - 2 618 178**
**DE - A - 2 651 786**
**GB - A - 592 255**
**GB - A - 1 244 551**

**IBM TECHNICAL DISCLOSURE BULLETIN, Band 22, Nr. 7, Dezember 1979, Seiten 2827-2828 New York, U.S.A. J.A. ZUMBADO: "Full aperture beamformer delay line" ACOUSTICAL HOLOGRAPHY, Band 5, 1974, ausgeg. durch P.S. Green Plenum Press New York, London J.F. HAVLICE et al.: "An electronically focused acoustic imaging device"**

(73) Patentinhaber: **SIEMENS AKTIENGESELLSCHAFT, Berlin und München Wittelsbacherplatz 2, D-8000 München 2 (DE)**

(72) Erfinder: **Sachs, Bertram, Dorfstrasse 30, D-8520 Erlangen (DE)**
Erfinder: **Borburgh, Jaques, Dr. Dipl.-Ing., Eichenstrasse 10, D-8521 Poxdorf (DE)**
Erfinder: **Feigt, Ingmar, Dr. Dipl.-Phys., Strümpellstrasse 22, D-8520 Erlangen (DE)**

ACTORUM AG

## Beschreibung

Die Erfindung bezieht sich auf ein Ultraschall-Array mit einer Anordnung der Wandlerelemente in Zeilen und Spalten, wobei die Wandlerelemente an gegenüberliegenden Kontaktflächen kontaktiert und sowohl den Kontakten der einen Kontaktfläche als auch den Kontakten der anderen Kontaktfläche Steuerelemente zugeordnet sind, die zur Einstellung vorwählbarer Sende- und/oder Empfangsflächen aus Wandlerelementen während eines Sende-/Empfangs-Zyklus, insbesondere zum Zweck der dynamischen Fokussierung, dienen.

Ultraschall-Arrays mit Umschaltmöglichkeit für Sende- und/oder Empfangsflächen sind beispielsweise als Einzelzeilen-Array aus der DE-C 24 43 686 und als Mehrzeilen-Array aus der DE-B 26 43 918 bekannt, von der auch die Erfindung ausgeht. Bei einem solchen Array könnte in üblicher Art jedem einzelnen Wandlerelement als Steuerelement ein eigener Schalter, insbesondere ein einzelner Transistorschalter, sowohl für die Heiss- als auch für die Erdseite zugeordnet werden. Dies würde jedoch in unnötiger Weise den Gesamtaufwand an Steuerelementen oder Schaltern erhöhen. Ausserdem erlaubt die spezielle Art des Schaltens mittels Transistorschaltern beim Aufbau unterschiedlicher Sende- und/oder Empfangsflächen lediglich ein diskontinuierliches Zuschalten von einzelnen Wandlerelementen zur gewünschten Fläche. Ein kontinuierliches Zuschalten von Einzelelementen im Sinne der Erzeugung einer kontinuierlichen variablen Apertur ist nicht vorgesehen; ein Einsatz kontinuierlich variabler Schaltelemente würde bei der benötigten Gesamtzahl aller Steuerelemente oder Schalter zu einem allzu hohen technischen Aufwand führen.

Aus der GB-A 1 244 551 ist bereits ein Ultraschall-Array bekannt, bei dem jedes Wandlerelement im Kreuzungspunkt einer zweidimensionalen Matrix angeordnet ist. Eine Schicht aus piezoresistivem Material ist zwischen zwei Sätze aus jeweils zueinander parallelen Kontaktierungsstreifen angeschlossen, wobei die beiden Sätze rechtwinklig zueinander liegen, so dass sich die zweidimensionale Matrix ergibt. Die Kontaktierungsstreifen sind gleichzeitig Leitungsverbindungen zu den benachbarten Wandlerelementen. Jedem Wandlerelement ist eine Eigenkapazität zugeordnet, die immer dann aufgeladen wird, wenn das betreffende Wandlerelement durch horizontale und vertikale Abtastpulse abgetastet wird, die gleichzeitig an die dem Wandlerelement zugeordnete Zeile und Spalte gelegt werden. Auf die Anordnung und Ausbildung von Schaltern wird in dieser Literaturstelle ebensowenig eingegangen wie auf die Variation von Sende- und Empfangsflächen und damit eine Veränderung der Apertur.

Aufgabe der vorliegenden Erfindung ist es, ein Ultraschall-Array der eingangs genannten Art anzugeben, das mit einer geringen Zahl von Steuerelementen auskommt und für die Untersuchung verschiedener Untersuchungstiefen mit geringem technischen Aufwand eine kontinuierlich variable Apertur der Sende u./od. Empfangsflächen ermöglicht.

Die Aufgabe wird erfindungsgemäss dadurch gelöst, dass die Wandlerelemente mit ihrer einen Kontaktfläche in Richtung der Zeilen zu Zeilengruppen und mit ihrer anderen Kontaktfläche in Richtung der Spalten zu Spaltengruppen zusammenkontaktiert sind, dass jeder Zeilengruppe höchstens ein einzelnes gemeinsames Zeilengruppen-Steuerelement und jeder Spaltengruppe höchstens ein einzelnes gemeinsames Spaltengruppen-Steuerelement zugeordnet ist, und dass die Zeilen- und/oder Spaltengruppen-Steuerelemente zumindest teilweise als kontinuierlich variable Widerstandselemente ausgebildet sind, die zum Zweck eines kontinuierlichen Aufbaus der Empfangs- und/oder Sendeflächen ein kontinuierliches Zuschalten einzelner Wandlerelemente zu diesen Flächen ermöglichen, so dass sich auf diese Weise eine kontinuierlich variable Apertur ergibt.

Bei der Erfindung ist die Menge der insgesamt benötigten Steuerelemente verhältnismässig klein. Der technische Aufwand an zugehörigen Steuerbausteinen ist damit ebenfalls verhältnismässig gering. Dies ermöglicht den angestrebten Übergang auf die kontinuierlich variable Apertur mit Hilfe der kontinuierlich variablen Widerstandselemente, insbesondere in Form von gesteuerten Widerständen. Als kontinuierlich steuerbare Widerstandselemente können steuerbare Feldeffekttransistoren, steuerbare Dioden o. dgl. dienen. Die im Betrieb kontinuierlich variierte Apertur sorgt dafür, dass für jede Untersuchungstiefe die Nahfeld-Fernfeld-Grenze für den vorgegebenen Anwendungszweck optimal genutzt werden kann. Ein sprunghaftes Umschalten von einer Tiefe auf andere Tiefen ist nicht mehr gegeben.

Die kontinuierlich variierte Apertur, insbesondere die im Empfangsfalle kontinuierlich wachsende Apertur, führt darüber hinaus zu einer Schalldruckverteilung mit Mittenzentrierung, insbesondere nach einem Gaussprofil. In einer solchen Schalldruckverteilung sind Schallnebenkeulen weitgehend unterdrückt; ein störender Einfluss von Schallnebenkeulen, insbesondere auf die Lateralauflösung, ist somit kaum mehr gegeben (s. Krautkrämer: «Werkstoffprüfung mit Ultraschall», 3 Auflage, Springer-Verlag 1975, Seiten 82 und 83).

Weitere Vorteile der Erfindung ergeben sich aus den Unteransprüchen. Hier ist insbesondere eine Ausgestaltung des Ultraschall-Arrays mit einem zusätzlichen, an Erde liegenden Kontaktbelag von Bedeutung. Dieser schirmt einfallende Hochfrequenz ab. Der Kontaktbelag verhindert den Einfall elektromagnetischer Strahlung (EMS) im Falle geöffneter, an Erde liegende Steuerelemente, d.h. bei Nichtvorhandensein eines Erdanschlusses.

Die Erfindung wird im nachfolgenden anhand von Ausführungsbeispielen in Verbindung mit der Zeichnung näher erläutert. Es zeigen:

Fig. 1 den Prinzipaufbau eines Mehrzeilen-Arrays,

Fig. 2 eine erste Anwendungsform im Prinzipschaltbild, bei der kontinuierlich variable Widerstandselemente eingesetzt werden,

Fig. 3 eine erste Anwendungsform im Prinzipschaltbild, bei der ebenfalls ein kontinuierlich variables Widerstandselement eingesetzt wird, und

Fig. 4 den Schichtaufbau eines Ultraschall-Arrays, bei dem variable Widerstandselemente zwecks Erzeugung einer kontinuierlich variablen Apertur bevorzugt eingesetzt werden können.

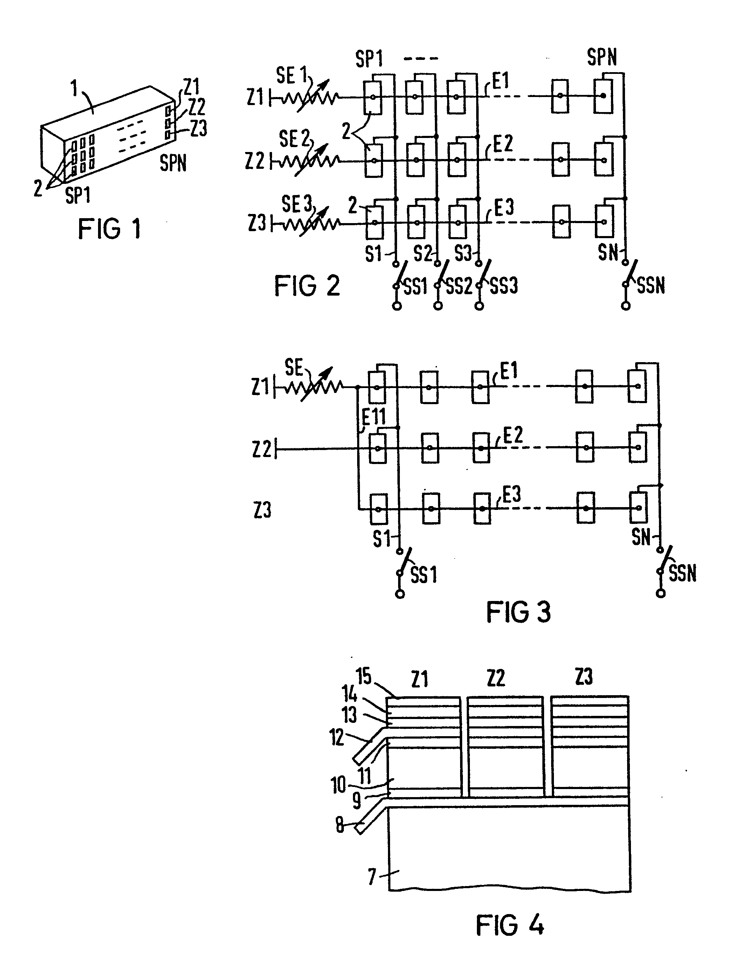

In der Figur 1 weist der Array-Trägerkörper 1 an seiner Applikationsfläche Ultraschall-Wandlerelemente 2 auf. Die Wandlerelemente 2 sind dabei in Spalten SP1 bis SPN und in Zeilen Z1, Z2 und Z3 angeordnet. Die Zahl der Zeilen beträgt im vorliegenden Fall 3. Die Zahl N der Spalten SP1 bis SPN ist sehr viel grösser, sie liegt beispielsweise bei 80 bis 120.

Die Figur 2 zeigt eine erste Ausführungsform einer gruppenweisen Kontaktierung von Wandlerelementen entlang den Spalten und den Zeilen. Die Spaltenkontaktbahnen sind mit S1, S2, S3... bis SN bezeichnet. Die Elementanschlüsse für die Spaltenkontaktbahnen S1 bis SN befinden sich jeweils auf der Rückseite der zugehörigen Wandlerelemente 2. Die Spaltengruppen-Steuerelemente für die Spaltenkontaktbahnen S1 bis SN sind mit SS1, SS2, SS3... bis SSN bezeichnet. Sie sind hier als Schalter ausgebildet. Die Zeilenkontaktbahnen sind jeweils mit E1 bis E3 angegeben. Die Kontaktierung der Wandlerelemente 2 entlang den Zeilen Z1, Z2, Z3 erfolgt, wie eingezeichnet, an der Vorderseite. Die Zeilengruppen-Steuerelemente sind mit SE1 bis SE3 bezeichnet. Sie sind im vorliegenden Falle kontinuierlich variable Widerstandelemente (kontinuierlich steuerbare Widerstände) und liegen an Erde. Bei den gesteuerten Widerstandelementen SE1 bis SE3 kann es sich auch um steuerbare Feldeffekttransistoren, steuerbare Dioden o. dgl. handeln. Die Spaltengruppen-Steuerelemente SS1 bis SSN sind die auf Potential liegenden Steuerelemente (= «Heiss»-Steuerelemente für die Sendeenergie im Sendefall bzw. für die Empfangsenergie im Empfangsfall.

Die Funktionsweise des Prinzipschaltbildes der Figur 2 ist die, dass zum Zwecke des Sendens eine vorwählbare Zahl von Spaltengruppen-Steuerelementen, z.B. die drei Spaltengruppen-Steuerelemente SS1 bis SS3, zu Beginn einer jeden Abtastung zusammen mit den drei Zeilen SE1 bis SE3 stromleitend gemacht bzw. geschlossen wird. Nach Aussenden eines Sendeimpulses durch die im vorliegenden Falle beispielweise aus neun Einzelementen gebildete Sendefläche werden z. B. die beiden Zeilengruppen-Speicherelemente SE1 und SE3 für die beiden äusseren Zeilen Z1 und Z2 wieder in den stromsperrenden Zustand gebracht. Bei weiterhin stromleitendem Zeilengruppen-Steuerelement SE2 und geschlossenen Spaltengruppen-Steuerelementen SS1 bis SS3 empfangen zuerst die drei Wandler-Elemente 2 der mittleren Zeile Z2 Echosignale; die Steuerelemente SE1 und SE3 der äusseren Zeilen werden erst um eine bestimmte Zeitdauer verzögert stromleitend. Somit wird also etwas verzögert auf grössere Empfangsfläche umgeschaltet. Auf diese Weis ergibt sich dynamische Fokussierung für den Empfangsfall.

Der beschriebene Vorgang wiederholt sich nun sukzessive mit fortschreitender Weitertaktung des Ultraschallstrahles in Richtung der Zeilen Z1 bis Z3. Dies geschieht in der üblichen Weise dadurch, dass nach jedem Sende-/Empfangszyklus für den nächstfolgenden der Sende-/Empfangsblock durch Schliessen eines nächstfolgenden Spaltengruppen-Steuerelements bei gleichzeitiger Öffnung des jeweils ersten dieses Blockes um eine Spalte weitergetaktet wird.

Das beschriebene Schaltschema ist jedoch lediglich exemplarisch. Modifikationen sind in jeder Beziehung möglich. So wird beispielweise auch im Sendefall dafür gesorgt werden, dass einzelne Steuerelemente eines Blockes zu unterschiedlichen Zeiten stromleitend gemacht oder geschlossen werden. Hierdurch ist dynamische Fokussierung im Sendefall möglich.

Sowohl die Spaltengruppen-Steuerelemente SS1 bis SSN als auch die Zeilengruppen-Steuerelemente SE1 bis SE3 könnten prinzipiell normale Transistorschalter sein, wie sie üblicherweise in der Array-Technik eingesetzt werden. Dieser Weg wird hier jedoch nicht beschritten. Von Vorteil ist es nämlich, wenn zumindest ein Teil dieser Steuerelemente als kontinuierlich variable Schaltelemente ausgebildet ist. Hierdurch ergibt sich die Möglichkeit des Überganges auf kontinuierlich variierbare Apertur. Die Fokusanpassung erfolgt dann dynamisch ohne Sprünge; die sich hierbei ergebende Schalldruckverteilung unterdrückt optimal Schallnebenkeulen.

Das Prinzipschaltbild der Figur 3 zeigt eine Vereinfachung des Schaltbildes der Figur 2 dahingehend, dass die beiden äusseren Zeilen Z1 und Z3 über eine Querkontaktierung E11 querverbunden sind. Die mittlere Zeile Z2 liegt direkt an Erdpotential. Aufgrund der Querkontaktierung E11 ist für die beiden äusseren Zeilen Z1, Z3 nur noch ein einziges Zeilengruppen-Steuerelement SE nötig. Auch dieses Zeilengruppen-Steuerelement SE ist ein kontinuierlich steuerbares Widerstandselement.

Figur 4 zeigt schliesslich den Schichtaufbau eines Ultraschall-Arrays in Kontaktierung entsprechend der Figur 2 (oder auch Figur 3 bei entsprechender Querkontaktierung). Gemäss der Darstellung in Figur 4 folgt auf einen Dämpfungskörper 7 eine Schicht aus spaltenweise angeordneten Kontaktbahnen 8, die als Spaltenkontaktbahnen S1 bis SN dienen und an die die Spaltengruppen-Steuerelemente SS1 bis SSN anschliessbar sind. In nächster Schichtung folgen dann die aus je einem Piezo-Element 10 mit rückseitiger und vorderseitiger Kontaktierung 9 bzw. 11 gebildeten Wandlerelemente. In weiterer Schicht folgen in Zeilen angeordnete Kontaktbahnen 12, die als Zeilenkontaktbahnen E1 bis E3 (Schalterde) dienen und an denen die Zeilengruppen-Steuerelemente SE1 bis SE3 (oder bei entsprechender Querkontaktierung SE) anschliessbar sind. Schliesslich folgen dann in weiterer Schichtung entlang den Zeilen über Anpassungsschichten 13 Schichten 14 für eine Schirmerde gegen HF-Einstrahlung und darauf wieder Schichten 15 für applikationsseitige Isolation.

Sämtliche Ausführungsbeispiele haben lediglich beispielhaften Charakter; es sind beliebige Modifikationen möglich, z. B. dahingegend, dass beliebig unterschiedliche Zahlen von Zeilen und Spalten gewählt werden. Das Array kann beispielsweise auch quadratische Form besitzen, nämlich dann, wenn die Zahl der Zeilen gleich der Zahl der Spalten ist. Die Grösse der einzelnen zu schaltenden Sende- und/oder Empfangsflächen kann durch entsprechend unterschiedliche Antsteuerung einzelner Spalten- und Zeilengruppen-Steuerelemente beliebig unterschiedlich (z. B. auch nach Programm) gewählt werden.

Die Aufteilung kann auch so sein, dass entlang den Spalten und/oder auch den Zeilen jeweils immer eine beliebige Zahl von Wandlerelementen zu Gruppen zusammenkontaktiert wird, so dass also auch innerhalb der Spalten und/oder Zeilen beliebige Gruppenansteuerungen über entsprechend zugeordnete Gruppen-Steuerelemente möglich sind.

## Patentansprüche

1. Ultraschall-Array mit einer Anordnung von Wandlerelementen (2) in Zeilen (Z1 bis Z3) und Spalten (SP1 bis SPN), wobei die Wandlerelemente (2) an gegenüberliegenden Kontaktflächen kontaktiert und sowohl den Kontakten (9) der einen Kontaktfläche als auch den Kontakten (11) der anderen Kontaktfläche Steuerelemente (SE1 bis SE3; SE; SS1 bis SSN) zugeordnet sind, die zur Einstellung vorwählbarer Sende- und/oder Empfangsflächen aus Wandlerelementen (2) während eines Sende-/Empfangszyklus, insbesondere zum Zwecke der dynamischen Fokussierung, dienen, dadurch gekennzeichnet, dass die Wandlerelemente (2) mit ihrer einen Kontaktfläche in Richtung der Zeilen (Z1 bis Z3) zu Zeilengruppen und mit ihrer anderen Kontaktfläche in Richtung der Spalten (SP1 bis SPN) zu Spaltengruppen zusammenkontaktiert sind, dass jeder Zeilengruppe höchstens ein einzelnes gemeinsames Zeilengruppen-Steuerelement (SE1 bis SE3; SE) und jeder Spaltengruppe höchstens ein einzelnes gemeinsames Spaltengruppen-Steuerelement (SS1 bis SSN) zugeordnet ist, und dass die Zeilen- und/oder Spaltengruppen-Steuerelemente zumindest teilweise als kontinuierlich variable Widerstandselemente ausgebildet sind, die zum Zwecke eines kontinuierlichen Aufbaus der Empfangs- und/oder Sendeflächen ein kontinuierliches Zuschalten einzelner Wandlerelemente (2) zu diesen Flächen ermöglichen, so dass sich auf diese Weise eine kontinuierlich variable Apertur ergibt.

2. Ultraschall-Array nach Anspruch 1, dadurch gekennzeichnet, dass als kontinuierlich variable Widerstandselemente (SE1 bis SE3; SE) gesteuerte Widerstände oder steuerbare Widerstandselemente wie steuerbare Feldeffekttransistoren oder steuerbare Dioden vorgesehen sind.

3. Ultraschall-Array nach Anspruch 1 oder 2, dadurch gekennzeichnet, dass in der Zeilen- und Spaltenkonfiguration entweder nur die Spaltengruppen-Steuerelemente oder nur die Zeilengruppen-Steuerelemente auf Potential liegen, während die zugehörigen Zeilengruppen-Steuerelemente bzw. Spaltengruppenelemente an Erde liegende Steuerelemente sind, und dass diese an Erde liegenden Steuerelemente die besagten kontinuierlich variablen Widerstandselemente sind.

4. Ultraschall-Array nach einem der Ansprüche 1 bis 3, dadurch gekennzeichnet, dass in symmetrischer Anordnung eine vorgegebene Zahl von Zeilen (Z2) direkt mit Erdpotential verbunden ist und eine weitere vorgegebene Zahl von Zeilen (Z1 und Z3) miteinander querverbunden ist, wobei diese querverbundenen Zeilen (Z1 und Z3) über ein einzelnes Steuerelement (SE) mit Erdpotential verbunden sind, das als variables Widerstandselement ausgeführt ist.

5. Ultraschall-Array nach einem der Ansprüche 3 oder 4, dadurch gekennzeichnet, dass zusätzlich zu den Kontaktanschlussbelägen für die an Erde liegenden Steuerelemente (SE1 - SE3; SE) kapazitiv gekoppelt auch noch ein Hochfrequenz-Einstrahlung abschirmender Kontaktbelag (14) vorhanden ist, der ständig auf Erdpotential liegt.

6. Ultrasschall-Array nach Anspruch 5, dadurch gekennzeichnet, dass in Schichtbauweise der abschirmende Kontaktbelag (14) unter Zwischenanordnung einer Anpassungsschicht (13) auf eine für die an Erde liegenden Steuerelemente vorgesehene Kontaktbahn (12) folgt.

7. Ultraschall-Array nach Anspruch 5 oder 6, dadurch gekennzeichnet, dass applikationsseitig auf dem abschirmenden Kontaktbelag (14) ein isolierender Belag (15) angeordnet ist.

8. Ultraschall-Array nach einem der Ansprüche 5 bis 7, dadurch gekennzeichnet, dass es schichtweise wie folgt aufgebaut ist:

a) auf einen Dämpfungskörper (7) folgt eine Schicht aus spaltenweise angeordneten Kontaktbahnen (8), die als Spaltenkontaktbahnen (S1 bis SN) und zum Anschluss an die Spaltengruppen-Steuerelemente (SS1 bis SSN) dienen;

b) auf die Kontaktbahnen (8) folgen in nächster Schichtung aus Piezo-Elementen (10) mit rückseitiger und vorderseitiger Kontaktierung (9, 11) ausgebildete Wandlerelemente;

c) auf die rückseitige Kontaktierung (11) folgen in weiterer Schicht in Zeilen angeordnete Kontaktbahnen (12), die als Zeilenkontaktbahnen (E1 bis E3) dienen und an denen die Zeilengruppen-Steuerelemente anschliessbar sind; und

d) unter weiterer Schichtung entlang der Zeilen (Z1 bis Z3) folgen über Anpassungsschichten (13) Schichten (14) für die Schirmerde gegen Hochfrequenzeinstrahlung und daran anschliessend Schichten (15) für eine applikationsseitige Isolation (Figur 4).

## Claims

1. Ultrasonic array with an arrangement of transducer elements (2) in lines (Z1 to Z3) and columns (SP1 to SPN), the transducer elements (2) being contacted to opposite contact surfaces and there being coordinated with both the contacts (9) of the one contact surface and the contacts (11) of the other contact surface control elements (SE1 to SE3; SE; SS1 to SSN) which serve to set preselectable emitting and/or receiving surfaces consisting of transducer elements (2) during one emitting/receiving cycle, more particularly for the purpose of dynamic focusing, characterised in that the transducer elements (2) are contacted together with their one contact surface in the direction of the lines (Z1 to Z3) to form line groups and with their other contact surface in the direction of the columns (SP1 to SPN) to form column groups, in that there is coordinated with each group of lines at most one sigle common line group control element (SE1 to SE3; SE) and with each group of columns at most one single common column group control element (SS1 to SSN), and in that the line and/or column group control elements are constructed at least partly as continuously variable resistance elements which for the purpose of continuous creation of the emitting and/or receiving surfaces allow continuous connection of individual transducer elements (2) to these surfaces so that a continuously variable aperture is thereby produced.

2. Ultrasonic array according to claim 1, characterised in the controlled resistances or controllable resistance elements such as controllable field effect transistors or controllable diodes are provided as continuously variable resistance elements (SE1 to SE3; SE).

3. Ultransonic array according to claim 1 or 2, characterised in that in the line and column configuration either only the column group control elements or only the line group control elements are connected to potential, while the associated line group control elements or column group elements respectively are earthed control elements, and in that these earthed control elements are said continuously variable resistance elements.

4. Ultrasonic array according to one of claims 1 to 3, characterised in that in a symmetrical arrangement a prescribed number of lines (Z2) are connected directly to earth potential, and a further preconnected, these cross-connected lines (Z1 and Z3) being connected by way of an individual control element (SE) to earth potential, which is constructed as a variable resistance element.

5. Ultrasonic array according to one of claims 3 and 4, characterised in that a contact coating (14) which shields high frequency irradiation and which is connected constantly to earch potential is capacitively coupled in addition to the contact connection coatings for the earthed control elements (SE1 - SE3; SE).

6. Ultrasonic array according to claim 5, characterised in that in the layer structure the shielding contact coating (14) follows a contact path (12) provided for the control elements connected to earth, with the interposition of a matching layer (13).

7. Ultrasonic array according to claim 5 or 6, characterised in that an insulating coating (15) is arranged on the application side on the shielding contact coating (14).

8. Ultrasonic array according to one of claims 5 to 7, characterised in that it is constructed in layers as follows:

a) a layer of contact paths (8) arranged in columns serving as column contact paths (S1 to SN) and for connection to the column group control elements (SS1 to SSN), follow an attenuation body (7);

b) in the next layer, transducer elements consisting of piezoelectric elements (10) with front and rear contacting (9, 11) follow the contact paths (8);

c) in a further layer, contact paths (12) arranged in lines, which serve as line contact paths (E1 to E3) and to which the line group control elements may be connected, follow rear contacting (11); and

d) with further layering along the lines (Z1 to Z3) follow over matching layers (13), layers (14) for earth shielding against high frequency irradiation and layers (15), connected thereto, for application-side insulation.

**Revendications**

1. Réseau à ultrasons comportant un agencement d'éléments transducteurs (2) en lignes (Z1 à Z3) et colonnes (SP1 à SPN), cependant que les éléments transducteurs sont mis en contact au niveau de surfaces de contact opposées et qu'aussi bien aux contacts (9) d'une des surfaces de contact qu'également aux contacts (11) de l'autre surface de contact sont associés des éléments de commande (SE1 à SE3; SE; SS1 à SSN) qui servent au réglage de surfaces d'émission et/ou de réception, pouvant être présélectionnées, provenant d'éléments transducteurs (2), pendant un cycle d'émission/réception, notamment dans le but de la focalisation dynamique, caractérisé par le fait que les éléments transducteurs (2) sont mis en contact les uns avec les autres par une de leurs surfaces de contact suivant la direction des lignes (Z1 à Z3) pour former des groupes de lignes et par leur autre surface de contact suivant la direction des colonnes (SP1 à SPN) pour former des groupes de colonnes, qu'à chaque groupe de lignes est associé au maximum un élément de commande commun individuel (SE1 à SE3; SE) de groupe de lignes et à chaque groupe de colonnes est associé au maximum un élément de commande commun individuel (SS1 à SSN) de groupe de colonnes, et que les éléments de commande de groupe de lignes et/ou de colonnes se présentent au moins partiellement sous la forme d'éléments résistifs qui peuvent varier de façon continue et qui, dans le but d'une réalisation continue des surfaces de réception et/ou d'émission, permettent une mise en circuit continue d'éléments résistifs avec ces surfaces de sorte qu'on obtienne de cette manière une ouverture qui peut varier de façon continue.

2. Réseau à ultrasons suivant la revendication 1, caractérisé par le fait qu'il est prévu des résistances commandées ou des éléments résistifs pouvant être commandés, tels que des transistors à effet de champ commandables ou des diodes commandables, en tant qu'éléments résistifs qui peuvent varier de façon continue (SE1 à SE3; SE).

3. Réseau à ultrasons suivant la revendication 1 ou 2, caractérisé par le fait que dans la configuration des lignes et des colonnes soit seuls les éléments de commande de groupe de colonnes soit seuls les éléments de commande de groupe de lignes se trouvent au potentiel, tandis que les éléments de commande de groupe de lignes ou les éléments de groupe de colonnes associées sont des éléments de commande se trouvant à la terre, et que ces éléments de commande se trouvant à la terre sont les dits éléments résistifs qui peuvent varier de façon continue.

4. Réseau à ultrasons suivant l'une des revendications 1 à 3, caractérisé par le fait que suivant un agencement symétrique, un nombre prédéterminé de lignes (Z2) est relié directement au potentiel de la terre et un autre nombre prédéterminé de lignes (Z1 et Z3) sont reliées transversalement les unes aux autres, ces lignes (Z1 et Z3) reliées transversalement étant reliées au potentiel de la terre par l'intermédiaire d'un élément de commande individuel (SE) qui est réalisé sous la forme d'un élément résistif variable.

5. Réseau à ultrasons suivant l'une des revendications 3 ou 4, caractérisé par le fait qu'en plus des couches d'établissement de contact pour les éléments de commande (SE1 - SE3; SE) reliés à la terre il est encore également prévu, avec un couplage capacitif, une couche de contact (14) de protection vis-à-vis d'un rayonnement haute fréquence, qui se trouve en permanence au potentiel de la terre.

6. Réseau à ultrasons suivant la revendication 5, caractérisé par le fait que suivant le mode de réalisation par couches la couche de contact de protection (14) suit une voie de contact (12) prévue pour les éléments de commande se trouvant à la terre, avec interposition d'une couche d'adaptation (13)

7. Réseau à ultrasons suivant la revendication 5 ou 6, caractérisé par le fait qu'une couche isolante (15) est disposée sur la couche de contact de protection (14) du côté d'application.

8. Réseau à ultrasons suivant l'une des revendications 5 à 7, caractérisé par le fait qu'il est réalisé par couches de la façon suivante:

a) à un corps d'amortissement (7) fait suite une couche de voies de contact (8) disposées en colonnes qui servent de voies de contact de colonne (S1 à SN) et au raccordement avec les éléments de commande (SS1 à SSN) de groupe de colonnes;

b) aux voies de contact (8) font suite, dans l'empilage suivant, des éléments transducteurs constitués par des piézoéléments (10) comportant un contact (9, 11) sur la face arrière et sur la face avant;

c) au contact (11) sur la face arrière font suite, dans une autre couche, des voie de contact (12) disposées en lignes qui servent de voies de contact de ligne (E1 à E3) et auxquelles peuvent être raccordés les éléments de commande de groupe de lignes; et

d) en formant un autre empilage suivant les lignes (Z1 à Z3) suivent, par l'intermédiaire de couches d'adaptation (13), des couches (14) pour la terre de blindage vis-à-vis d'un rayonnement haute fréquence et des couches (15) qui s'y raccordent pour un isolement du côté d'application (figure 4).

0 036 577

FIG 1

FIG 2

FIG 3

FIG 4

7